# EUROPEAN PATENT APPLICATION

(11) **EP 2 574 294 A1**
(43) Date of publication of application: **03.04.2013**
(21) Application number: 12181383.6
(22) Date of filing: 22.08.2012
(51) Int. Cl.: A61B 17/3207

(54) **Rotating occlusion treatment system**

(30) Priority: 30.09.2011 US 201113250174
(71) Applicant: Covidien LP, Mansfield, MA 02048 (US)
(72) Inventor: Lee, Stephen, San Jose, CA 95120 (US); Mahli, Arnaz, Watertown, MA 02472 (US)
(74) Representative: Gray, James

(57) **Abstract**

An occlusion treatment device for the treatment of a body lumen that is at least partially blocked by an occlusion. The occlusion treatment device includes a catheter and a debriding mechanism that is configured and dimensioned for axial movement through the catheter and is extendable beyond a distal end of the catheter. The debriding mechanism includes a head assembly with a debriding tip having an outer surface with at least one aperture formed therein, wherein at least one of the apertures is complemented by a corresponding debriding member that extends outwardly from the outer surface of the debriding tip and directs debris from the occlusion through the at least one aperture to facilitate removal of the debris from the lumen. The debriding mechanism is rotatable to effect debriding of the occlusion.

## Description

### BACKGROUND

### 1. Technical Field

The present disclosure relates to systems, apparatus, and methods for the treatment of occlusions within a lumen, such as a blood vessel, and more specifically to rotating occlusion treatment systems and corresponding methods of use.

### 2. Background of Related Art

Apparatus and methods used to establish, or maintain, patency in an occluded lumen are well known in the art. For example, it is known that occlusions can be treated through the delivery of a thrombolytic agent, such as a tissue plasminogen activator (tPA). Thrombolytic agents, however, are expensive, require lengthier hospital procedures, and create risks of drug toxicity and bleeding complications as the occlusions are broken down. Additionally, it is known that the delivery of non-mechanical energy, such as RF, microwave, or ultrasonic energy, can be utilized to treat occlusions, but these systems carry the risk of damaging healthy tissues collateral to those at the treatment site. It is also known that occlusions can be treated mechanically via the application of force to the occlusion, such as, for example, through the use of a cutting assembly.

During the treatment of an occlusion with a mechanical system, as the occlusion is debrided, debris is created that may be subject to circulation through the patient's blood stream, potentially resulting in distal embolization of the debris. Many systems incorporate, or are used in conjunction with, supplemental mechanisms, such as filters or aspiration means, in order to prevent such circulation of debris. Also, to make sure the debris is captured, mechanical devices tend to cut slowly to enable the supplemental mechanisms to capture the debris and not be overwhelmed.

Despite the number of systems known in the art, there remains a need for an occlusion treatment system of simpler design that is capable of quickly and safely debriding an occlusion, and aspirating any corresponding debris, in order to establish, and/or maintain, patency in a partially or wholly occluded lumen.

### SUMMARY

In one aspect of the present disclosure, an occlusion treatment device is disclosed that is configured and dimensioned for the treatment of a lumen that is at least partially blocked by an occlusion. The occlusion treatment device includes a catheter with a body portion having proximal and distal ends, and a debriding mechanism that is configured and dimensioned for axial movement through the catheter such that the debriding mechanism is extendable beyond the distal end of the body portion of the catheter.

The debriding mechanism is rotatable to effect debriding of the occlusion, and includes a head assembly with a debriding tip. The debriding tip includes an outer surface with at least one aperture formed therein complemented by a corresponding debriding member that extends outwardly from the outer surface of the debriding tip. Each debriding member is configured and dimensioned to direct debris from the occlusion through the at least one aperture to facilitate removal of the debris from the lumen.

It is envisioned that the outer surface may include a plurality of apertures, wherein at least one of the plurality of apertures includes the corresponding debriding member, and that the debriding tip may have a substantially hemispherical configuration to maximize the volume of material removed from the occlusion during debriding.

The debriding mechanism may further include a rotatable torque sleeve that is operatively connected to the head assembly, whereby rotational motion of the torque sleeve is transmitted to the head assembly to cause corresponding rotation of the head assembly. It is also envisioned that an outer sheath may be positioned about the torque sleeve.

In order to operatively connect the torque sleeve to the head assembly, in one embodiment of the disclosure, the head assembly further includes a coupling section positioned between the torque sleeve and the head assembly.

The coupling section may include a proximal portion, an intermediate portion secured to the proximal portion, and a distal portion secured to the intermediate portion. It is envisioned that the proximal, intermediate, and distal portions of the coupling section may be monolithically formed. Additionally, it is envisioned that the proximal portion of the coupling section may be welded to the torque sleeve, and that the head assembly may be welded to the distal portion of the coupling section.

In one embodiment of the coupling section, the proximal portion of the coupling section defines a substantially uniform outer transverse cross-sectional dimension, the intermediate portion of the coupling section defines a non-uniform outer transverse cross-sectional dimension, which, for example, increases in a distal direction, and the distal portion of the coupling section defines a substantially uniform outer transverse cross-sectional dimension. In this embodiment, it is envisioned that the outer transverse cross-sectional dimension of the distal portion of the coupling section may be greater than the outer transverse cross-sectional dimension of the proximal portion of the coupling section.

The debriding mechanism may further include a fluid supply tube that is in fluid communication with a fluid suction and supply device in order to communicate a fluid beyond a distal end of the body portion of the catheter, and remove the fluid together with the debris created during debriding of the occlusion from the lumen.

Additionally, it is envisioned that the catheter may include at least one expandable element that is secured to the body portion of the catheter.

In another aspect of the present disclosure, an occlusion treatment system is disclosed that includes a fluid suction and supply device, a catheter having a body portion with proximal and distal ends, and a debriding mechanism that is configured and dimensioned for axial movement through the catheter such that the debriding mechanism is extendable beyond the distal end of the body portion of the catheter.

The debriding mechanism may include a rotatable torque sleeve, and a head assembly that is operatively connected to the torque sleeve such that rotation of the torque sleeve causes corresponding rotation of the head assembly, wherein the head assembly includes a debriding tip with an outer surface having at least one aperture formed therein. It is envisioned that each aperture in the outer surface of the debriding tip may be complemented by a corresponding debriding member extending outwardly from the outer surface of the debriding tip, and that each debriding member may be configured and dimensioned to direct debris created during debriding of the occlusion through the at least one aperture.

The debriding mechanism is in fluid communication with the fluid suction and supply device such that the debris created during debriding of the occlusion is aspirated from the lumen through the torque sleeve.

It is envisioned that the debriding tip may have a substantially hemispherical configuration corresponding to an internal contour of the lumen to maximize the volume of material removed from the occlusion during debriding.

It is further envisioned that the head assembly may also include a coupling section that operatively connects the torque sleeve to the head assembly. For example, the coupling section may be positioned between the torque sleeve and the head assembly.

In yet another aspect of the present disclosure, a method is disclosed for treating a lumen that is at least partially blocked by an occlusion. The method includes the steps of positioning a catheter within the lumen, advancing a debriding mechanism through the catheter such that a debriding tip of the debriding mechanism is positioned adjacent the occlusion, effecting rotation of the debriding tip, whereby a debriding member on an outer surface of the debriding tip debrides the occlusion, and directs debris from the occlusion through a corresponding aperture in the outer surface of the debriding tip, and aspirating the debris through the debriding mechanism to remove the debris from the lumen.

### BRIEF DESCRIPTION OF THE DRAWINGS

Various embodiments of the present disclosure are described herein with reference to the drawings wherein:
FIG. 1 is a schematic view illustrating of one embodiment of an occlusion treatment system;
FIG. 2 is a side, perspective view of a catheter and the debriding mechanism of FIG. 1;
FIG. 3 is a transverse, cross-sectional view taken through lines 3-3 in FIG. 1;
FIG. 4 is a longitudinal, cross-sectional view taken through lines 4-4 in FIG. 1;
FIG. 5 is a side view illustrating a head assembly of the debriding mechanism of FIG 1;
FIG. 6 is a partial, schematic view illustrating a catheter of the presently disclosed occlusion treatment device seen in FIG. 1 positioned within the lumen prior to insertion of the debriding mechanism;
FIG. 7 is a partial, schematic view illustrating the presently disclosed occlusion treatment device of FIG. 6 with the debriding mechanism advanced through the catheter;
FIGS. 8 and 9 are partial, schematic views of the presently disclosed occlusion treatment device, of FIG. 6 illustrating use of the debriding mechanism;
FIG. 10 is a partial, schematic view illustrating an alternative embodiment of a catheter of the presently disclosed occlusion treatment device; and
FIG. 11 is a partial, schematic view illustrating the catheter seen in FIG. 10.

### DESCRIPTION OF THE EMBODIMENTS

Embodiments of the presently disclosed occlusion treatment system will now be described in detail with reference to the drawings wherein like reference numerals identify similar or identical elements. As used herein, the term "distal" refers to that portion of the presently disclosed occlusion treatment system, or component thereof, that is furthest from the user during proper use, while the term "proximal" refers to that portion of the occlusion treatment system, or component thereof, that is closest to the user during proper use. Additionally, the term "lumen" should be understood to include any lumen within the body, either natural or artificial, such as, for example, blood vessels, blood vessel grafts, fistulas, and the like. Moreover, the term "occlusion" should be understood to encompass any partial, or total, blockage of a lumen, such as, for example, thrombus, atheromas, plaque, and the like. Finally, as used herein below, the term "debride" should be understood to include various mechanical methods of treating an occlusion, including, but not limited to abrading, softening, cutting, debulking, grinding, and the like.

Throughout the following description, well known functions and constructions are not described in detail so as to avoid obscuring the present disclosure in unnecessary detail.

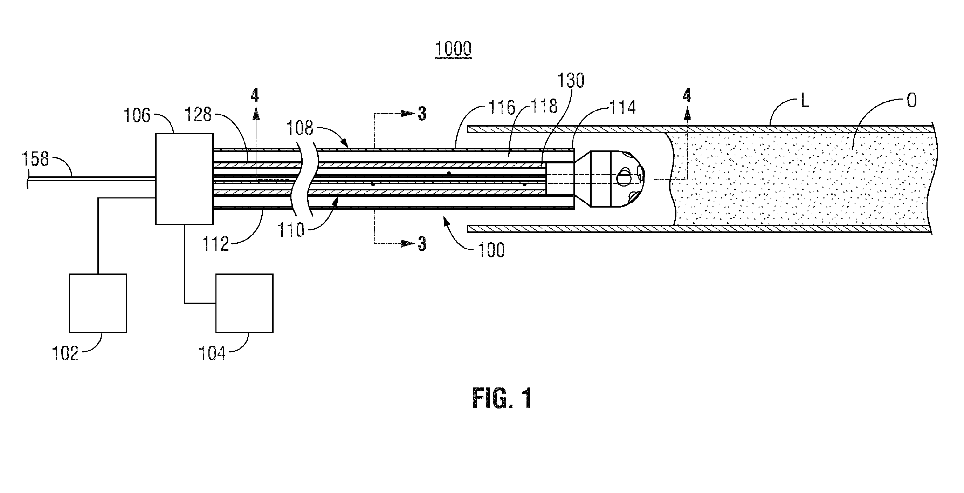

FIG. 1 illustrates an occlusion treatment system 1000 according to one embodiment of the present disclosure for treatment of a lumen L that is at least partially blocked by an occlusion O. The occlusion treatment system 1000 includes an occlusion treatment device 100, a source of fluid 102, for example saline, a fluid suction/supply device 104, and a control unit 106.

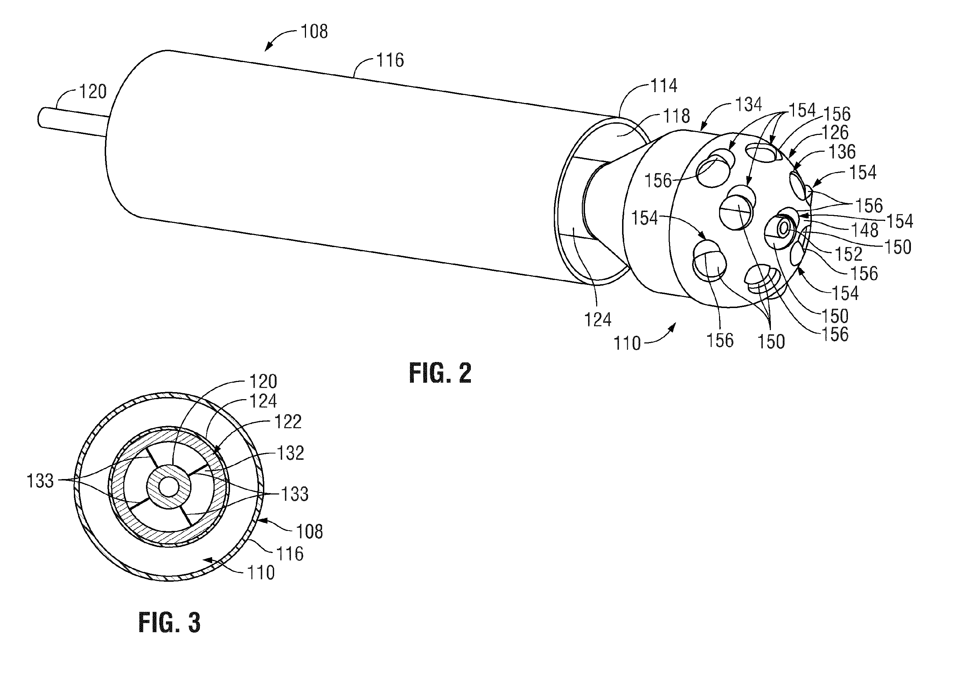

With reference to FIGS. 2-5 as well, the occlusion treatment device 100 includes a catheter 108, and a debriding mechanism 110.

The catheter 108 includes a proximal end 112 that is in communication with the control unit 106 (FIG. 1), an open distal end 114, and a body portion 116 with a passageway 118 that extends therethrough. The catheter 108 may be formed from any suitable biocompatible material sufficiently pliable to facilitate insertion of the catheter 108 into the lumen L. Suitable materials include, but are not limited to, polymeric materials, elastomeric materials, for example, silicone and fabric materials, or synthetic resins, for example, polyurethane, polyether block amide such as Pebax™, polyimides, PEEK and fluorinated ethylene propylene (FEP).

It is envisioned that the body portion 116 of the catheter 108 may also include one or more ports (not shown) formed therein so as to facilitate the distribution of a treatment agent to the occlusion O, such as a thrombolytic agent, for example, a tPA.

With particular reference to FIGS. 2-4, the debriding mechanism 110 will be discussed. The debriding mechanism 110 may include a fluid supply tube 120, a torque sleeve 122, an outer sheath 124, and a head assembly 126.

The fluid supply tube 120 may be operatively connected to the fluid source 102 (FIG. 1) and the fluid suction/supply device 104, for example, through the control unit 106, whereby a fluid, such as saline, can be communicated through the debriding mechanism 110 to the occlusion O (FIG. 1). The fluid supply tube 120 may be formed from any biocompatible material suitable for the intended purpose of communicating fluid to the occlusion O. For example, it is envisioned that the fluid supply tube 120 may be formed from thermoplastic materials, such as polyetherether-ketone (PEEK), as well as Pebax™, polyimides and FEP.

The torque sleeve 122 is configured, dimensioned, and adapted to impart rotational motion to the debriding mechanism 110 in a manner that will be described below, and may be formed from any biocompatible material suited for this intended purpose. For example, it is envisioned that the torque sleeve 122 may be formed from stainless steel, nitinol, tungsten and titanium, although the use of other materials is envisioned.

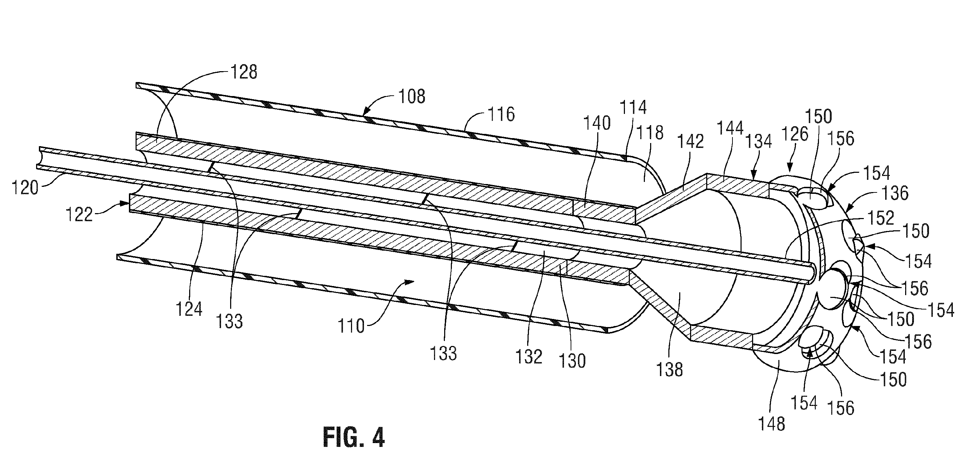

The torque sleeve 122 includes respective proximal and distal ends 128, 130 (FIGS. 1, 4), and defines a longitudinal channel 132 that extends therebetween. As best seen in FIGS. 3 and 4, the fluid supply tube 120 may be positioned within the channel 132, and connected to the torque sleeve 122 such that rotation of the torque sleeve 122 causes corresponding rotation of the fluid supply tube 120, whereby the torque sleeve 122 and the fluid supply tube 120 are rotated in unison. It is envisioned that the torque sleeve 122 and the fluid supply tube 120 may be connected together using any attachment means, such as adhesives, welds, pins, rivets, or the like. For example, as best seen in FIGS. 3 and 4, the supply tube 120 and the torque sleeve 122 may be connected by one or more attachment members 133. While the attachment members 133 are illustrated as being positioned within the channel 132, the configuration and dimensions of the attachment members 133 are such that the attachment members 133 do not substantially inhibit the communication of fluid through the channel 132.

The proximal end 128 (FIG. 1) of the torque sleeve 122 is operatively connected to the fluid suction/supply device 104, for example, through the control unit 106. During debriding of the occlusion O, through the application of a vacuum force created by the fluid suction/supply device 104, fluid and/or debris can be removed from the site of the occlusion O through the channel 132, as will described in further detail below.

The outer sheath 124 may be positioned coaxially about the torque sleeve 122 (FIGS. 3 and 4) in a manner permitting the torque sleeve 122 to rotate within the outer sheath 124 without causing rotation of the outer sheath 124. The outer sheath 124 thus provides a barrier between the inner wall W of the lumen L and the rotational components of the debriding mechanism 110, thereby substantially minimizing the likelihood of inadvertent damage to the lumen L. In an alternate embodiment, the outer sheath 124 may be formed of a heat shrinkable material, such as FEP, which is formed over torque sleeve 122, such that the outer sheath 124 provides a barrier between channel 132 and debriding mechanism 110 to protect the non-rotating catheter 108.

With continued reference to FIGS. 2-5, the head assembly 126 will be discussed. The head assembly 126 is connected to the distal end 130 (FIG. 4) of the torque sleeve 122, and includes a coupling section 134, and a tip member 136.

The coupling section 134 operatively connects the tip member 136 to the torque sleeve 122 such that rotational movement of the torque sleeve 122 is imparted to the tip member 136, whereby the torque sleeve 122 and the tip member 136 are rotated in unison. The coupling section 134 may be formed from any biocompatible material suitable for the intended purpose of operatively connecting the tip member 136 to the torque sleeve 122 including, but not limited to, **stainless steel, tungsten and nitinol.**

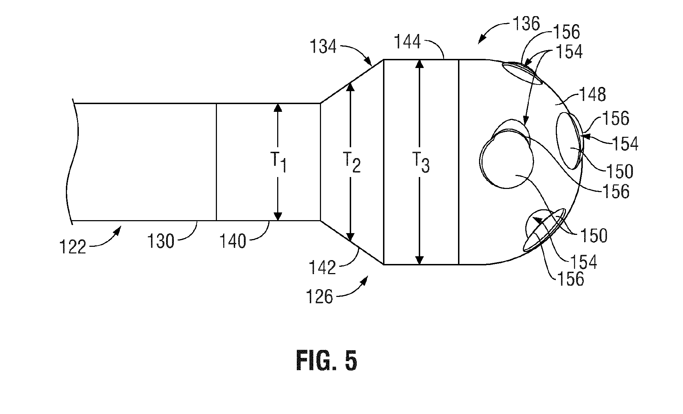

The coupling section 134 may include a hollow interior 138, as well as respective proximal, intermediate, and distal portions 140, 142, 144. In one specific embodiment of the coupling section 134, which is illustrated in FIG. 5, the proximal portion 140 defines a substantially uniform transverse cross-sectional dimension T₁, the intermediate portion 142 defines a non-uniform transverse cross-sectional dimension T₂, and the distal portion 144 defines a substantially uniform transverse cross-sectional dimension T₃. Specifically, as seen in FIG. 5, the transverse cross-sectional dimension T₁ of the proximal portion 140 is less than the transverse cross-sectional dimension T₂ of the intermediate portion 142, which gradually increases in the distal direction, and the transverse cross-sectional dimension T₂ of the intermediate portion 142 is less than the transverse cross-sectional dimension T₃ of the distal portion 144. Although the debriding mechanism 110 is illustrated, and described, as including the embodiment of the coupling section 134 seen in FIG. 5, it should be understood that, in alternative embodiments of the present disclosure, the specific configuration of the coupling section 134 may be altered or varied without departing from the scope of the present disclosure.

It is envisioned that the respective proximal, intermediate, and distal portions 140, 142, 144 of the coupling section 134 may be monolithically formed. Alternatively, however, it is envisioned that the respective proximal, intermediate, and distal portions 140, 142, 144 of the coupling section 134 may constitute discrete structures that are secured together through any suitable attachment means, such as, for example, adhesives, welds, pins, rivets, or the like.

The proximal portion 140 of the coupling section 134 is secured to the torque sleeve 122, and the distal portion 144 of the coupling section 134 is secured to the tip member 136. It is envisioned that the coupling section 134 may be secured to the torque sleeve 122 and the tip member 136 through any suitable attachment means, such as, for example, adhesives, welds, pins, rivets, or the like. In one specific embodiment, however, the proximal portion 140 of the coupling section 134 is laser-welded to the distal end 130 of the torque sleeve 122, and the distal portion 144 of the coupling section 134 is laser-welded to the tip member 136. Alternatively, the torque sleeve 122 and the coupling section 134 may be integrally formed, such as by molding.

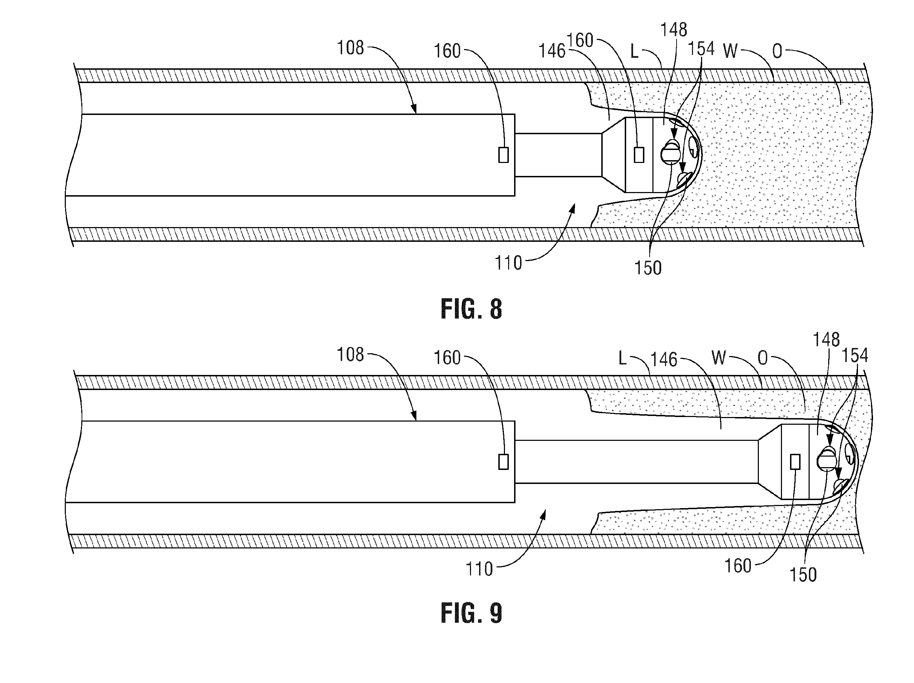

With continued reference to FIGS. 2-5, the tip member 136 will be discussed. The tip member 136 is configured, dimensioned, and adapted to debride, and penetrate, the occlusion O in order to form an opening 146 therein, as seen in FIGS. 8 and 9. The tip member 136 may be formed from any biocompatible material, including, but not limited to, stainless steel, tungsten and nitinol. Further, the tip member 136 may assume any geometric configuration, suitable for this intended purpose. In one embodiment, the tip member 136 may be substantially hemispherical in configuration, as illustrated in the specific embodiment seen in FIGS. 2-5, whereby the configuration of the tip member 136 corresponds to the internal contour of the lumen L in order to maximize the volume of material removed from the occlusion O during debriding. It should be appreciated, however, that the configuration and dimensions of the tip member 136 may be altered or varied in alternative embodiments without departing from the scope of the present disclosure.

The exemplary tip member 136 includes an outer surface 148 with one or more apertures 150 formed therein. As best seen in FIG. 4, at least one aperture 150 is in communication with a distal end 152 of the fluid supply tube 120, which permits the communication of fluid through the tip member 136 to the occlusion O. In those embodiments of the tip member 136 including multiple apertures 150, such as the embodiment seen in FIGS. 2-5, it is envisioned the apertures 150 may be spaced circumferentially, and axially, from each other in a predetermined manner so as to avoid overlapping portions, and the creation of ridges that may result in uneven debriding of the occlusion O.

Each aperture 150 is complemented by a debriding member 154 that extends outwardly from the outer surface 148 of the tip member 136. The debriding members 154 are configured and dimensioned in order to debride the occlusion O, and direct debris through the apertures 150, and thus, into the hollow interior 138 (FIG. 4) of the coupling section 134, as will be discussed in further detail below. In one specific embodiment of the tip member 136, which is shown in FIGS. 2-5, it is envisioned that each debriding member 154 may include an incisive cutting edge 156 to further enhance the ability of the tip member 136 to debride the occlusion O.

With reference now to FIG. 1 in particular, the control unit 106 will be described. The control unit 106 may regulate operation of the occlusion treatment device 100, as well as the other components of the occlusion treatment system 1000, and thus, may include various, motors microprocessor, electronic components, software, and/or firmware components. Software may be provided in a machine-readable medium storing executable code and/or other data in order to facilitate processing of user-specific data.

One specific function of the control unit 106 may be to provide the user with feedback from the occlusion treatment device 100, and/or information pertaining to environmental conditions, operating parameters, etc. The control unit 106 may be configured to output operational information concerning such feedback and information to the user. For example, the control unit may be configured to monitor the position of the tip member 136 (FIG. 2), the rotational velocity of the tip member 136, the temperature at the site of the occlusion O (FIG. 1), the infusion rate and/or the volume of fluid being provided to the site of the occlusion O, the aspiration rate and/or the volume of fluid and debris being withdrawn from the site of the occlusion O, the elapsed time of the procedure, or any other physical parameters contemplated.

It is further envisioned that the control unit 106 may implement certain automated, and/or selectable, control features. For example, various routines or programs including operating parameters may be preselected and stored in the control unit 106 to be selectable by the user, thereby permitting the user to input specified parameters/data to effect appropriate operation of the device. Thus, according to one embodiment of the disclosure, the control unit 106 may control features, and/or operation, of the occlusion treatment device 100 based on the data or information input by the user. For example, the user may input data concerning the occlusion O, such as the dimensions of the occlusion O, the type of tissue comprising the occlusion O, the rate of blood flow, the volume of blood flow, the percentage of restriction in the lumen L, the type of lumen L that is occluded, for example, a blood vessel, the location of the lumen L, the particular dimensions of the lumen L, the desired advance rate of the occlusion treatment device 100, the desired infusion and/or aspiration rates, the desired rotational velocity of the tip member 136 (FIG. 2), etc. Based on the data input by the user, the control unit 106 may calculate and implement automated operating conditions in order to automatically regulate, for example, the rotational velocity of the tip member 136. Various operating parameters, operating conditions, patient conditions, and the like may also be recorded and stored within the control unit 106 so as to preserve a record of the patient, and the details of the procedure.

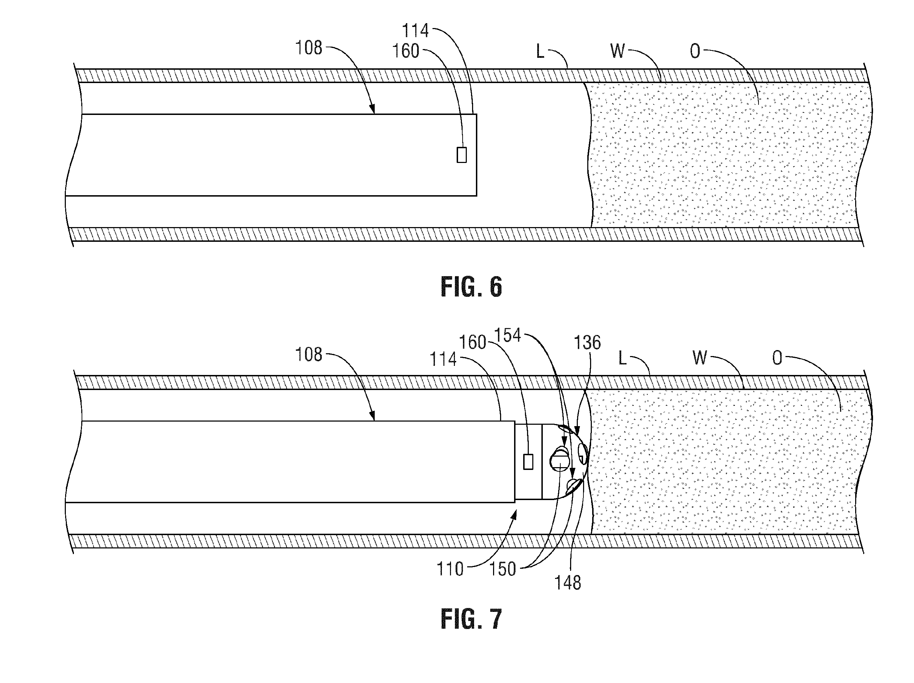

An exemplary method of treating the occlusion O with the occlusion treatment system 1000 will now be discussed with reference to FIGS. 6-9. Initially, the catheter 108 is inserted into the lumen L, and is advanced until the distal end 114 of the catheter 108 is positioned substantially adjacent to the occlusion O. It is envisioned that positioning of the catheter 108 may be aided through the use of a guidewire 158 (FIG. 1) coaxially positionable within the catheter 108. If utilized, a distal end of the guidewire 158 is initially positioned within the lumen L, for example, through the use of a needle cannula (not shown), as is known in the art. Thereafter, the guidewire 158 is advanced to a desired location, either adjacent, or beyond, the occlusion O, and a proximal end of the guidewire 158 is inserted into the distal end 114 of the catheter 108 such that the catheter 108 can be advanced distally over the guidewire 158. A dilator/sheath assembly (not shown) may also be used to further facilitate insertion of the occlusion treatment device 100 into the lumen L.

In an alternative method of use, it is envisioned that a guide catheter (not shown) may be inserted into the lumen L over the guidewire 158, and that the catheter 108 may be subsequently advanced through the guide catheter into the desired position.

Following positioning of the catheter 108 in the desired manner, the debriding mechanism 110 is inserted into, and advanced through, the catheter 108 until the tip member 136 extends beyond the distal end 114 of the catheter 108, and is positioned adjacent the occlusion O.

Thereafter, fluid is communicated from the fluid source 102 to the site of the occlusion O through the fluid supply tube 120 of the debriding mechanism 110 via the fluid suction/supply device 104. The torque sleeve 122, and thus, the coupling section 134 and the tip member 136, are then caused to rotate. Rotation and axial advancement of the tip member 136 causes repeated engagement of the debriding member(s) 154 and the cutting edge(s) 156 (FIG. 2) with the occlusion O, thereby cutting into, and debriding, the occlusion O in order to create the opening 146 in the occlusion O seen in FIGS. 8 and 9. As used herein, the term "rotation" should be understood to include continuous rotation about a longitudinal axis of the torque sleeve 122, as well as oscillation or rotation in alternating (different) directions about the longitudinal axis of the torque sleeve 122.

In various embodiments of use, it is envisioned that rotation of the debriding mechanism 110 may be either manually controlled by the user, or alternatively, that rotation of the debriding mechanism 110 may be effected by a drive mechanism (not shown) that is operatively connected to the control unit 106 so as to automate rotation of the debriding mechanism 110.

During debridement of the occlusion O, debris can be aspirated from the lumen L using the fluid suction/supply device 104 (FIG. 1), as described above. Specifically, upon the application of a vacuum force created by the fluid suction/supply device 104, debris from the occlusion O is drawn through the apertures 150 and proximally through the channel 132 (FIGS. 3 and 4) of the torque sleeve 122 and out of the debriding mechanisms 110. By removing debris from the lumen L, patency of the lumen L can be increased while maintaining visualization at the site of the occlusion O, and minimizing the likelihood of distal embolization of the debris. Further, by removing debris all the way out of the proximal end of the debriding mechanism 110, there is no need to remove the debriding mechanism 110 from the catheter 108 to remove the debris. In other words, debris may be continually removed from the body lumen and the debriding mechanism 110.

In order to further facilitate treatment of the occlusion O, additional mechanical force can be applied to the occlusion O via reciprocal axial movement of the debriding mechanism 110 relative to the occlusion O.

Debriding of the occlusion O continues until the tip member 136 penetrates the occlusion O. Thereafter, the catheter 108 can be advanced through the occlusion O in order to facilitate further treatment, for example, through the placement of a stent, or the delivery of a therapeutic agent. In order to facilitate advancement of the catheter 108 through the lumen L, and the opening 146 (FIGS. 8 and 9) in the occlusion O, it is envisioned that one or more components of the occlusion treatment system 1000 may include a lubricous coating.

During the course of the procedure in which the occlusion treatment system 1000 is employed, it is envisioned that the positions of the catheter 108, and/or the debriding mechanism 110, may be monitored using a visualization system. For example, the catheter 108 and/or the debriding mechanism 110 may include one or more markers 160, as seen in FIGS. 6-9, such as radiopaque markers, that can be viewed by the user on a monitor, or the like.

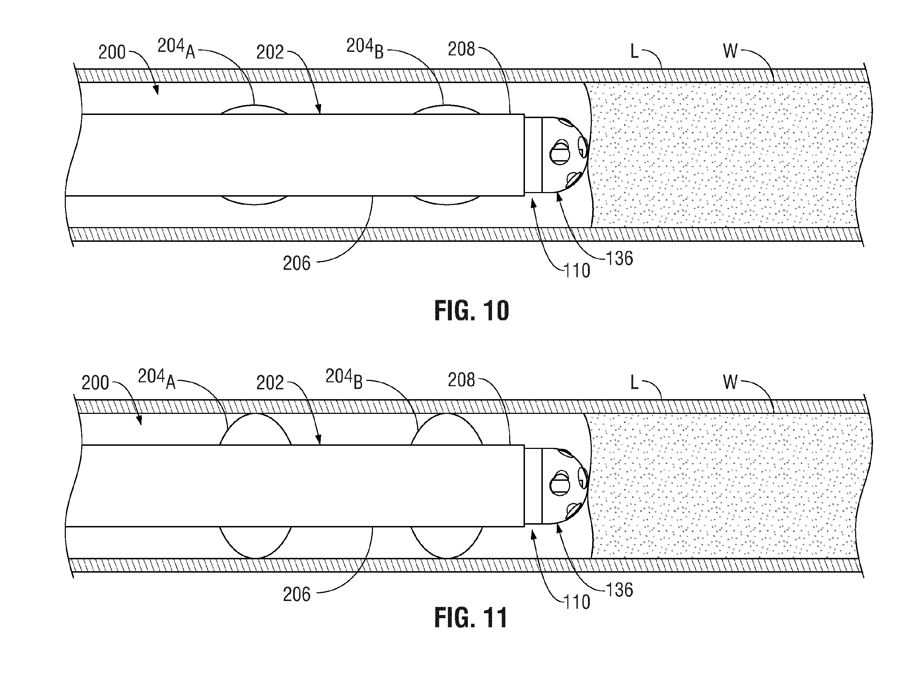

With reference now to FIGS. 10 and 11, an alternative embodiment of the catheter 108, which is identified by the reference character 200, will be discussed in connection with use of the debriding mechanism 100 (FIGS. 1-9). The catheter 200 is substantially similar to the aforedescribed catheter 108, and as such, will only be discussed with respect to any differences therefrom.

The catheter 200 includes a body portion 202 with one or more expandable elements 204_{A}, 204_{B} that are secured to an outer surface 206 thereof. Although illustrated as including expandable elements 204_{A} and 204_{B}, it should be understood that alternative embodiments of the catheter 200 may include either greater, or fewer, numbers of expandable elements dependent upon the requirements of the particular procedure in which the occlusion treatment system 1000 is employed.

The expandable elements 204_{A}, 204_{B} are in communication with a fluid source, such as, for example, the fluid source 102 (FIG. 1), such that the expandable elements 204_{A}, 204_{B} are movable between an initial position (FIG. 10), wherein the expandable elements 204_{A}, 204_{B} define first transverse cross-sectional dimensions, and a subsequent position (FIG. 11), wherein the expandable elements 204_{A}, 204_{B} define second, larger transverse cross-sectional dimensions. In the initial position, the smaller transverse cross-sectional dimensions of the expandable elements 204_{A}, 204_{B}, which may correspond to the outer transverse cross-sectional dimension of the body portion 202, facilitate insertion of the catheter 200 into the lumen L, and movement of the catheter 200 through the lumen L. In the subsequent position, the enlarged transverse cross-sectional dimensions of the expandable elements 204_{A}, 204_{B} facilitate engagement with an internal wall W of the lumen L in order to center the catheter 200 within the lumen L, and maintain the catheter 200 in a desired position. By centering the catheter 200 within the lumen L, the internal wall W of the lumen L can be separated from the tip member 136 of the debriding mechanism 110 in order to prevent inadvertent damage to the lumen L. For example, by expanding the expandable elements 204_{A}, 204_{B}, and centering the catheter 200 within the lumen L, the tip member 136 may be separated from the internal wall W of the lumen L by a predetermined distance of, for example, approximately 1.5 mm to approximately 2 mm.

During use of the catheter 200, following positioning of the catheter 200 in the desired manner, i.e., such that a distal end 208 of the catheter 200 is positioned adjacent the occlusion O, the debriding mechanism 110 is inserted into, and advanced through, the catheter 200 until the tip member 136 extends beyond the distal end 208 of the catheter 108, and is positioned adjacent the occlusion O, as seen in FIG. 10.

Thereafter, the expandable elements 204_{A}, 204_{B} are expanded, as seen in FIG. 11, in order to properly orient, and maintain, the position of the catheter 200, and thus, the debriding mechanism 110, within the lumen L. Fluid is then communicated from the fluid source 102 (FIG. 1) to the site of the occlusion O, and the tip member 136 is caused to rotate and axially advance/or oscillate in order to debride the occlusion O in the manner discussed above.

Further, once the tip member 136 has advanced through the occlusion, the expandable elements 204A, 204B may be deflated to advance the catheter 200 such that the occlusion is positioned within the expandable elements. Thereafter, the expandable elements 204a, 204b can be expanded to further treat the occlusion.

Through reference to the foregoing description, it should be understood that each embodiment of the presently disclosed occlusion treatment device is capable of mechanical and non-mechanical treatment of an occlusion. As indicated above, certain types of occlusive tissue are more suited to certain methods of removal. For example, chronic clots, as compared to acute clots, cannot be effectively removed using solely chemical agents, such as tPA. Accordingly, the treatment capabilities of the occlusion treatment device can be used alone, or in combination, with other capabilities to effectively remove an occlusion from a lumen. For example, where the occlusive tissue is a chronic clot, the clot can be effectively removed using the mechanical capabilities of the occlusion treatment device. As an additional example, it is not beyond the scope of the present disclosure to treat an occlusion mechanically via manipulation of the occlusion treatment device in the manner described above, as well as chemically through the delivery of a therapeutic agent.

Persons skilled in the art will understand that the devices and methods specifically described herein and illustrated in the accompanying drawings are non-limiting, exemplary embodiments. It is envisioned that the elements and features illustrated or described in connection with one exemplary embodiment may be combined with those of another embodiment without departing from the scope of the present disclosure. As well, one skilled in the art will appreciate further features and advantages of the presently disclosed occlusion treatment system based on the above-described embodiments. Accordingly, the present disclosure is not to be limited by what has been particularly shown and described, except as indicated by the appended claims.

## Claims

1. An occlusion treatment device configured and dimensioned for treatment of a lumen at least partially blocked by an occlusion, the occlusion treatment device comprising:
a catheter including a body portion having proximal and distal ends; and
a debriding mechanism configured and dimensioned for axial movement through the catheter such that the debriding mechanism is extendable beyond the distal end of the body portion of the catheter, the debriding mechanism including a head assembly with a debriding tip having an outer surface with at least one aperture formed therein complemented by a corresponding debriding member extending outwardly from the outer surface of the debriding tip, wherein the debriding mechanism is rotatable to effect debriding of the occlusion, and each debriding member is configured and dimensioned to direct debris from the occlusion through the at least one aperture to facilitate removal of the debris from the lumen.

2. An occlusion treatment device according to claim 1, wherein the outer surface of the debriding tip includes a plurality of apertures, at least one of the plurality of apertures including a corresponding debriding member extending outwardly from the outer surface of the debriding tip, the debriding member being configured and dimensioned to direct debris from the occlusion through the at least one aperture.

3. An occlusion treatment device according to claim 2, wherein each aperture in the outer surface of the debriding tip is complemented by a corresponding debriding member.

4. An occlusion treatment device according to any one of the preceding claims, wherein the debriding tip has a substantially hemispherical configuration corresponding to an internal contour of the lumen to maximize the volume of material removed from the occlusion during debriding.

5. An occlusion treatment device according to any one of the preceding claims, wherein the head assembly further includes a coupling section operatively connected to a torque sleeve of the debriding mechanism, the coupling section being positioned between the torque sleeve and the head assembly.

6. An occlusion treatment device according to claim 5, wherein the coupling section includes a proximal portion, an intermediate portion secured to the proximal portion, and a distal portion secured to the intermediate portion.

7. An occlusion treatment device according to claim 6, wherein the proximal, intermediate, and distal portions of the coupling section are monolithically formed.

8. An occlusion treatment device according to any one of claims 5 to 7, wherein the torque sleeve is configured and dimensioned for rotation such that rotational motion of the torque sleeve is transmitted to the head assembly to cause corresponding rotation of the head assembly.

9. An occlusion treatment device according to claim 8 further including an outer sheath positioned about the torque sleeve.

10. An occlusion treatment device according to claims 8 or 9, wherein the proximal portion of the coupling section is welded to the torque sleeve, and the head assembly is welded to the distal portion of the coupling section.

11. An occlusion treatment device according to any one of claims 6 to 10, wherein the proximal portion of the coupling section defines a substantially uniform outer transverse cross-sectional dimension.

12. An occlusion treatment device according to any one of claims 6 to 11, wherein the intermediate portion of the coupling section defines a non-uniform outer transverse cross-sectional dimension.

13. An occlusion treatment device according to claim 12, wherein the outer transverse cross-sectional dimension of the intermediate portion of the coupling section increases in a distal direction.

14. An occlusion treatment device according to any one of claims 6 to 13, wherein the distal portion of the coupling section defines a substantially uniform outer transverse cross-sectional dimension, the outer transverse cross-sectional dimension of the distal portion of the coupling section being greater than that of the proximal portion of the coupling section.

15. An occlusion treatment device according to any one of the preceding claims, wherein the debriding mechanism further includes a fluid supply tube in fluid communication with a fluid suction and supply device in order to communicate a fluid beyond a distal end of the body portion of the catheter, and remove the fluid together with the debris created during debriding of the occlusion from the lumen.
